# EUROPÄISCHE PATENTSCHRIFT

(11) **EP 0 951 561 B1**
(45) Veröffentlichungstag und Bekanntmachung des Hinweises auf die Patenterteilung: **08.08.2001**
(21) Anmeldenummer: 97927041.0
(22) Anmeldetag: 26.05.1997
(51) Int. Cl.: C12P 13/00

(54) **ENZYMATISCHE VERFAHREN ZUR HERSTELLUNG VON (S)-CYANHYDRINEN**
ENZYMATIC PROCESSES FOR PREPARING (S)-CYANOHYDRINS
PROCEDES ENZYMATIQUES POUR PREPARER DES (S)-CYANHYDRINES

(30) Priorität: 13.01.1997 AT 4197
(43) Veröffentlichungstag der Anmeldung: 27.10.1999
(73) Patentinhaber: DSM Fine Chemicals Austria GmbH, 4021 Linz (AT)
(72) Erfinder: KIRCHNER, Gerald, D-46485 Wesel (DE); WIRTH, Irma, A-4470 Enns (AT); WERENKA, Christian, A-4052 Ansfelden (AT); GRIENGL, Herfried, A-8047 Graz (AT); SCHMIDT, Michael, A-8113 St. Oswald (AT)
(74) Vertreter: Klostermann, Ingrid
(86) Internationale Anmeldenummer: EP9702692
(87) Internationale Veröffentlichungsnummer: WO9830711

(56) Entgegenhaltungen:
- EP-A- 0 539 767
- US-A- 5 346 816
- TETRAHEDRON LETTERS, Bd. 31, Nr. 9, 1990, Seiten 1249-1252, XP002039224 EFFENBERGER F. ET AL.: "Enzyme-Catalysed Synthesis of (S)-Cyanohydrins and Subsequent Hydrolysis to (S)-Alpha-hydroxy-carboxylic Acids" in der Anmeldung erwähnt
- J.BIOL.CHEM., Bd. 271, Nr. 10, 1996, Seiten 5884-5891, XP002022209 HASSLACHER M. ET AL.: "Molecular Cloning of the Full-length cDNA of (S)-Hydroxynitrile Lyase from Hevea brasiliensis" in der Anmeldung erwähnt
- TETRAHEDRON, Bd. 52, Nr. 23, 1996, Seiten 7833-7840, XP002039225 SCHMIDT M. ET AL.: "Preparation of Optically Active Cyanohydrins Using the (S)-Hydroxynitrile Lyase from hevea brasiliensis"

## Beschreibung

Cyanhydrine sind etwa zur Synthese von alpha-Hydroxysäuren, alpha-Hydroxyketonen, beta-Aminoalkohoien, die zur Gewinnung biologisch wirksamer Stoffe, z. B. pharmazeutischer Wirkstoffe, Vitamine oder auch pyrethroider Verbindungen Verwendung finden, von Bedeutung.

Die Herstellung eines Cyanhydrins kann durch Anlagerung einer Cyanidgruppe an den Carbonylkohlenstoff eines Aldehyds oder eines unsymmetrischen Ketons erfolgen, wobei Enantiomenrengemische optisch aktiver Cyanhydrine entstehen.

Da in einem biologisch wirksamen Enantiomerengemisch normalerweise nur eines der beiden Enantiomere biologisch wirksam ist, hat es nicht an Versuchen gefehlt, ein Verfahren zur Herstellung des (S)-Enantiomeren eines optisch aktiven Cyanhydrins in möglichst hoher optischer Reinheit zu finden.

So ist etwa in Makromol. Chem. 186, (1985), 1755-62 ein Verfahren zur Gewinnung von (S)-Cyanhydrinen durch Umsetzung von Aldehyden mit Blausäure in Gegenwart von Benzyloxycarbonyl-(R)-phenylalanin-(R)-histidinmethylester als Katalysator beschrieben. Die optische Reinheit der erhaltenen (S)-Cyanhydrine ist aber höchst unbefriedigend.

In EP-A-0 326 063 ist ein enzymatisches Verfahren zur Herstellung von optisch aktiven (R)- oder (S)-Cyanhydrinen durch Umsetzung von aliphatischen, aromatischen oder heteroaromatischen Aldehyden oder Ketonen mit Blausäure in Gegenwart von (R)-Oxynitrilase (EC 4.1, 2.10) aus Prunus amygdalis oder Oxynitrillase (EC 4.1, 2.11) aus Sorghum bicolor beschrieben. Beispiele für die stereospezifische Herstellung von aliphatischen (S)-Cyanhydrinen sind nicht angegeben. Dies ist nicht verwunderlich, denn in Angew. Chemie 102 (1990), Nr. 4, pp 423 - 425 ist von Erfindern, die in EP-A-0 326 063 genannt sind, angegeben, daß von der (S)-Oxynitrilase aus Sorghum bicolor keine aliphatischen (S)-Cyanhydrine mit Blausäure hergestellt werden können. Dieser Befund wird auch durch F. Effenberger et al. in Tetrahedron Letters Vol. 31 No. 9 (1990), pp. 1249 - 1252 bestätigt.

EP 0 632 130 beschreibt weiters ein Verfahren bei welchem aliphatische Aldehyde oder unsymmetrische aliphatische Ketone mit Blausäure und Oxynitrilase aus Hevea brasiliensis stereospezifisch zu (S)-Cyanhydrinen umgesetzt werden. Die Umsetzung erfolgt gemäß EP 0 632 130 bevorzugt in einem wässrigen Verdünnugsmittel ohne Zusatz organischer Lösungsmittel, da diese, wie in EP 0 632 130 beschrieben, die Aktivität des Enzyms rasch hemmen.

EP 0 539 767 beschreibt ein ähnliches Verfahren zur Herstellung von (S)-Cyanhydrinen, unter Verwendung spezieller Cyanidgruppendonoren anstelle von Blausäure. Auch EP 0 539 767 weist darauf hin, daß organische Lösungsmittel die Aktivität des Enzyms rasch hemmen.

In Tetrahedron Bd. 52, Nr. 23, 1996, S. 7833 - 7840 wird die Verwendung von rekombinanter Hydroxynitrillyase aus Hevea brasiliensis in einem wässrigen Puffersystem beschrieben.

Es wurde nun unerwarteterweise gefunden, daß die Verwendung von rekombinanter Hydroxynitrillyase (Hnl) aus Hevea Brasiliensis die Umsetzung einer Vielzahl von Carbonylverbindungen, wie etwa aliphatische, alicyclische, ungesättigte, aromatisch substituierte aliphatische, aromatische, sowie heteroaromatische Aldehyde und Ketone zu den entsprechenden Cyanhydrinen ermöglicht, wobei sich die rekombinante Hnl durch eine hohe Beständigkeit gegenüber organischen Lösungsmitteln auszeichnet.

Gegenstand der Erfindung ist daher ein Verfahren zur Herstellung des (S)-Enantiomeren eines optisch aktiven Cyanhydrins durch Umsetzung eines Aldehyds oder eines Ketons mit einem Cyanidgruppendonor, das dadurch gekennzeichnet ist, daß der Aldehyd oder das Keton in einem organischen Verdünnungsmittel in Gegenwart einer rekombinanten (S)-Hydroxynitrillyase aus Hevea brasiliensis mit einem Cyanidgruppendonor umgesetzt wird und das gebildete (S)-Cyanhydrin aus dem Reaktionsgemisch isoliert wird.

Als Ausgangsmaterialien im erfindungsgemäßen Verfahren werden ein Aldehyd oder ein Keton, ein Cyanidgruppendonor, eine rekombinante Hydroxynitrillyase und ein Verdünnungsmittel eingesetzt.

Unter Aldehyden sind dabei aliphatische, aromatische oder heteroaromatische Aldehyde zu verstehen. Unter aliphatischen Aldehyden sind dabei gesättigte oder ungesättigte aliphatische, geradkettige, verzweigte oder cyclische Aldehyde zu verstehen. Bevorzugte aliphatische Aldehyde sind geradkettige Aldehyde mit insbesondere 2 bis 18 C-Atomen, bevorzugt von 2 bis 12, die gesättigt oder ein- oder mehrfach ungesättigt sind. Der Aldehyd kann dabei sowohl C-C-Doppelbindungen als auch C-C-Dreifachbindungen aufweisen. Der Aldehyd kann unsubstituiert oder durch unter den Reaktionsbedingungen inerte Gruppen, beispielsweise durch gegebenenfalls substituierte Aryl- oder Heteroarylgruppen wie Phenyl- oder Indolylgruppen, durch Halogen-, Ether-, Alkohol-, Acyl-, Carbonsäure-, Carbonsäureester-, Nitro- oder Azidogruppen substituiert sein.
Beispiele für aromatische oder heteroaromatische Aldehyde sind Benzaldezyd bzw. verschieden substituierte Benzaldehyde wie etwa 3-Phenoxybenzaldehyd, weiters Furfural, Anthrazen-9-carbaldehyd, Furan-3-carbaldehyd, Indol-3-carbaldehyd, Naphthalin-1-carbaldehyde, Phthaldialdehyde, Pyrazol-3-carbaldehyd, Pyrol-2-carbaldehyd, Thiophen-2-carbaldehyd, Isophthalaldehyd oder Pyridinaldehyde u.s.w..
Ketone sind aliphatische, aromatische oder heteroaromatische Ketone, bei denen das Carbonylkohlenstoffatom gleich oder ungleich substituiert ist. Unter aliphatischen Ketonen sind gesättigte oder ungesättigte, geradkettige, verzweigte oder cyclische Ketone zu verstehen. Die Ketone können gesättigt oder ein- oder mehrfach ungesättigt sein. Sie können unsubstituiert, oder durch unter Reaktionsbedingungen inerte Gruppen, beispielsweise durch gegebenenfalls substituierte Aryl- oder Heteroarylgruppen wie Phenyl- oder Inolylgruppen, durch Halogen-, Ether-, Alkohol-, Acyl-, Carbonsäure-, Carbonsäureester-, Nitro- oder Azidogruppen substituiert sein.
Beispiele für aromatische oder heteroaromatische Ketone sind Acetophenon, Benzophenon u.s.w..
Bevorzugt werden Aldehyde und unsymmetrische Ketone umgesetzt.

Aldehyde und Ketone die sich für das erfindungsgemäße Verfahren eignen, sind bekannt oder wie üblich herstellbar.

Als Cyanidgruppendonor kommt Blausäure oder ein Cyanhydrin der allgemeinen Formel R₁R₂C(OH)(CN), in Betracht. In der Formel 1 bedeuten R₁ und R₂ unabhängig voneinander Wasserstoff oder eine unsubstituierte oder mit unter den Reaktionsbedingungen inerten Gruppen substituierte Kohlenwasserstoffgruppe, oder R₁ und R₂ gemeinsam eine Alkylengruppe mit 4 oder 5 C-Atomen, wobei R₁ und R₂ nicht gleichzeitig Wasserstoff bedeuten. Die Kohlenwasserstoffgruppen sind aliphatische oder aromatische, bevorzugt aliphatische Gruppen. Bevorzugt bedeuten R₁ und R₂ Alkylgruppen mit 1 bis 6 C-Atomen, ganz bevorzugt ist der Cyanidgruppendonor Acetoncyanhydrin.

Die Herstellung des Cyanidgruppendonors kann nach bekannten Verfahren erfolgen. Cyanhydrine, insbesonders Acetoncyanhydrin, sind auch käuflich zu erwerben.

Bevorzugt wird Blausäure oder Acetoncyanhydrin als Cyanidgruppendonor eingesetzt.
Als Hydroxynitrillyase wird rekombinante (S)-Hnl aus Hevea brasiliensis eingesetzt.
Geeignete rekombinante (S)-Hnl wird beispielsweise aus gentrechnisch modifizierten Mikroorganismen wie etwa Pichia pastoris oder Saccharomyces cerevisiae erhalten. Bevorzugt wird rekombinante (S)-Hnl aus Pichia pastoris eingesetzt.
Durch die funktionelle Überexpression in der methylotrophen Hefe Pichia pastoris kann diese Hnl in beliebiger Menge gewonnen werden. (M.Hasslacher et al., J. Biol. Chem. 1996, 271, 5884) Dieses Expressionssystem eignet sich besonders für Fermentationen mit hoher Zelldichte. So ist es möglich, etwa 20 g reines Enzym pro Liter Fermentationsmedium zu erhalten. Die erreichbaren spezifischen Aktivitäten des gereinigten rekombinanten Proteins sind etwa doppelt so hoch als jene des natürlichen Enzyms, welches aus den Blättern des Baumes Hevea brasiliensis isoliert wurde. Nach dem Zellaufschluß kann die cytosolische Fraktion ohne weitere Reinigung verwendet werden, wodurch der Arbeitsaufwand minimiert wird. Das Enzym ist nicht glykosyliert und besitzt auch keine prosthetische Gruppe, die bei Abspaltung vom Proteinteil zur Inaktivierung führen würde.
Die Hnl ist bei Raumtemperatur mehrere Tage ohne signifikanten Aktivitätsverlust einsetzbar, und ist bei -20°C ausreichend langzeitstabil. Dadurch ergibt sich die Möglichkeit der mehrmaligen Verwendung der selben Enzymcharge. Das Enzym zeichnet sich auch durch eine hohe Beständigkeit gegenüber Lösungsmitteln aus. Daher besteht die Möglichkeit, für die enzymatische Reaktion organische Solventien einzusetzen, was sich günstig auf die Produktivität des jeweiligen Prozesses auswirkt.

Die Hydroxynitrillyase kann gereinigt oder ungereinigt, als solche oder immobilisiert eingesetzt werden. Die Bereitstellung und Reinigung der Hydroxynitrillyase kann beispielsweise durch Fällung mit Ammoniumsulfat und anschließender Gelfiltration, etwa gemäß D. Selmar et al., Physiologia Plantarum 75 (1989), 97-101 erfolgen.

Die erfindungsgemäße Umsetzung erfolgt in einem organischen Verdünnungsmittel.
Als organische Verdünnungsmittel können mit Wasser nicht mischbare aliphatische oder aromatische Kohlenwasserstoffe, die gegebenenfalls halogeniert sind, Alkohole, Ether, Ester verwendet werden.

Bevorzugt werden Ethylacetat, Diisopropylether, Methyl-tert.butylether, Dibutylether verwendet.

Die (S)-Hnl kann dabei entweder immobilisiert in dem organischen Verdünnungsmittel vorliegen, die Umsetzung kann jedoch auch in eine Zweiphasensystem, mit nicht-immobilisierter (S)-Hnl erfolgen, wobei als organisches Verdünnungsmittel ein mit Wasser nicht mischbares Verdünnungsmittel wie etwa aliphatische oder aromatische Kohlenwasserstoffe, die gegebenenfalls halogenisiert sind, Ether oder Ester eingesetzt wird.

Pro g Aldehyd oder Keton werden etwa 50 bis 300 g Verdünnungsmittel und 200 bis 20 000 IU Aktivität Hydroxynitrillyase, bevorzugt etwa 500 bis 5 000 IU, zugesetzt. Ein IU (International Unit) drückt dabei die Bildung von einem Mikromol Produkt pro Minute und pro Gramm Enzymrohisolierung aus. Die benötigte Menge der jeweiligen Hydroxynitrillyase ermittelt man am besten in einem Aktivitätstest, etwa gemäß Selmar et al., Analytical Biochemistry 166 (1987), 208-211.

Pro Mol eingesetzte Aldehyd- oder Ketogruppe werden mindestens ein Mol, bevorzugt 1 bis 2 Mole Cyanidgruppendonor zugegeben.

Die Reaktionsmischung wird bei Temperaturen von etwa 0 °C bis zur Desaktivierungstemperatur der Hydroxynitrillyase, bevorzugt von 20 bis 30 °C geschüttelt oder gerührt.
Dabei wird die Cyanidgruppe vom Cyanidgruppendonor auf das Carbonylkohlenstoffatom des eingesetzten Aldehyds oder Ketons übertragen und es entsteht überwiegend das (S)-Enantiomere des, dem eingesetzten Aldehyd oder Keton entsprechenden, optisch aktiven Cyanhydrins. Der Fortschritt der Reaktion kann dabei unter anderem gaschromatographisch verfolgt werden.

Nach erfolgter Umsetzung kann das gebildete Cyanhydrin aus der Reaktionsmischung mit Hilfe eines organischen Lösungsmittels, das mit Wasser nicht mischbar ist, etwa aliphatische oder aromatische gegebenenfalls halogenierte Kohlenwasserstoffe, z.B. Pentan, Hexan, Benzol, Toluol, Methylenchlorid, Chloroform, Chlorbenzole, Ether wie etwa Diethylether, Diisopropylether oder Ester, beispielsweise Essigsäureethylester oder Mischungen solcher Lösungsmittel extrahiert werden. Sollte die Reinheit des extrahierten Produktes nicht ausreichend sein, kann eine Reinigungsoperation angeschlossen werden. Die Reinigung kann durch eine bekannte Methode erfolgen und gelingt am besten chromatographisch.

### Beispeil 1:

178 mg 3-Phenylpropanal (1,33 mmol) wurden in 2,8 ml Diisopropylether gelöst und die Lösung mit einem Kühlbad auf 0 - 5°C temperiert. Nach Zugabe von 1.1 ml wässriger Enzymlösung (900 lU/ml) ((S)-Hnl aus Pichia pastoris in 50 mM Phosphatpuffer) wurde 5 min bei 0 - 5°C gerührt und anschließend 200 µl wasserfreie Blausäure rasch auf einmal zugegeben. Das Reaktionsgefäß wurde druckdicht verschlossen und die Lösung 2 Stunden bei 0 - 5°C gerührt. Dreimalige Extraktion mit je 10 ml Diisopropylether, trocknen über wasserfreiem Natriumsulfat, abdestillieren des Lösungsmittels im Vakuum und säulenchromatographische Reinigung ergab das optisch aktive Cyanhydrin als einheitliche Substanz (204 mg farbloses Öl, 95 % isolierte Ausbeute), [α]_{D}²⁰ + 7,1° (c 1,3, CHCl₃). Zur Derivatisierung wurde das gereinigte Cyanhydrin in 10 ml trockenem Methylenchlorid aufgenommen und mit 2 Moläquivalenten Acetanhydrid und 2 Moläquivalenten Pyridin versetzt. Nach 10 Stunden Reaktionszeit bei Raumtemperatur wurde mit je 10 ml 5 % H₂SO₄, destilliertem Wasser und gesättigter Natriumhydrogencarbonat-Lösung gewaschen, die organische Phase über wasserfreiem Natriumsulfat getrocknet und das Lösungsmittel im Vakuum entfernt. Säulenchromatographische Reinigung ergab 221 mg (S)-(-)-2-Acetoxy-4-phenylbutannitril als farbloses Öl (86 % isolierte Ausbeute), [α]_{D}²⁰ - 43.4° (c 1,95, CHCl₃).
e.e = 94 % (gaschromatographisch bestimmt)
¹H-HMR: δ (ppm) 2,13 (s, 3H); 2,25 (q, 2H); 2,85 (t, 2H); 5.28 (t, 1H); 7,21-7,37 (m, 5H).
¹³C-NMR: 169,13; 139,31; 128,90; 128,48; 126,80; 116,89; 60,72; 33,89; 30,88; 20.34.

### Beispiel 2:

0,4 g mikrokristalline Cellulose wurden in 2,7 ml 0.02 M Natriumacetatpuffer 1 Stunde bei Raumtemperatur quellen gelassen und anschließend der wäßrige Puffer durch eine Filtration entfernt. Dem derart vorbehandelten Träger wurden 1,35 ml einer wässrigen Hydroxynitril Lyase (aus Pichia pastoris in 50 mM Phosphatpuffer) Lösung (390 lU/ml) zugesetzt und 10 min bei Raumtemperatur geschüttelt. Anschließend wurde diese Suspension am Rotationsverdampfer auf einen Wassergehalt von 40 % eingestellt. Das so erhaltene Enzymimmobilisat wurde mit 2,8 ml Ethylacetat (gesättig mit 0,01 M Natriumacetatpuffer, pH 5.4) und mit 141 mg frisch destilliertem Benzaldehyd (1,33 mmol) versetzt. Nach Zugabe von 5,25 mmol wasserfreier Blausäure (200 µl) wurde das Reaktionsgefäß druckdicht verschlossen und das Reaktionsgemisch 1 Stunde bei Raumtemperatur gerührt. Danach wurde das Enzymimmobilisat durch eine Friltration entfernt, dreimal mit je 5 ml Ethylacetat (gesättigt mit 0,01 M Natriumacetatpuffer, pH 5,4) nachgewaschen und über wasserfreiem Natriumsulfat getrocknet. Das Lösunsmittel wurde unter vermindertem Druck entfernt und das derart erhaltene rohe Cyanhydrin säulenchromatographisch gereinigt. Man erhielt 170 mg eines farblosen Öles (96 % isolierte Ausbeute), [α]_{D}²⁰-46.5° (c 1,4, CHCl₃).
Das reine Cyanhydrin wurde in 10 ml trockenem Methylenchlorid gelöst und mit 2 Moläquivalenten Acetanhydrid und 2 Moläquivalenten Pyridin versetzt. Nach Reaktion über Nacht wurde mit je 15 ml 5 % H₂SO₄, destilliertem Wasser und gesättigter Natriumhydrogencarbonat-Lösung extrahiert und über wasserfreiem Natriumsulfat getrocknet. Nach Entfernung des Lösungsmittels wurde die Substanz säulenchromatographisch gereinigt. Es wurden 221 mg (S)-2-Acetoxy-2-phenylacetonitril als farbloses Öl (99 % isolierte Ausbeute) erhalten.
[α]_{D}²⁰-7,24° (c 2,3; CHCl₃)
e.e >99 % (gaschromatographisch bestimmt).
¹H-NMR: δ (ppm) 2,16 (s, 3H); 6,42 (s, 1H); 7,48 (m, 5H).
¹³C-NMR: 168,87; 131,80; 130,48; 129,32; 127,94; 116,12; 62,92; 20,54.

### Beispiel 3:

300 IU Hydroxynitril Lyase aus Pichia pastoris wurden in einem flüssigkristallinen System, bestehend aus Di-n-butylether, Wasser und einem Tensid immobilisiert. Dieser Enzympräparation wurden bei 0 - 5°C 2 ml Di-n-butylether, 1 mmol Benzaldehyd (106 mg) und 3,94 mmol wasserfreie Blausäure (150 µl) zugesetzt. Das Gemisch wurde 3 Stunden bei 0 - 5°C gerührt, anschließend die organische Phase abgegossen und dreimal mit je 5 ml Di-n-butyiether nachgewaschen, Trocknen mit wasserfreiem Natriumsulfat ergab eine Lösung des rohen Cyanhydrins, welches direkt mit 2 Moläquivalenten Acetanhydrid und 2 Moläquivalenten Pyridin in das entsprechende Acetat übergeführt wurde. Nach 8 Stunden Reaktionszeit bei Raumtemperatur wurde die Lösung mit je 15 ml 5 % H₂SO₄, destilliertem Wasser und gesättigter Natriumhydrogencarbonat-Lösung gewaschen, mit wasserfreiem Natriumsulfat getrocknet und das Lösungsmittel unter vermindertem Druck entfernt. Die gaschromatische Analyse ergab eine Ausbeute von 73 % und einen Enantiomerenüberschuß von >99 %.

### Beispiel 4:

0,4 g Celite 545 wurden in 5ml 0,02 M Histidinpuffer (pH 6,8) suspendiert und 2 Stunden bei Raumtemperatur geschüttelt. Nach Entfernung des Quellmittels durch eine Filtration wurden 2,7 ml einer wässrigen Hydroxynitril Lyase-Lösung (390 lU/ml) aus Pichia pastoris zugetropft und 15 min bei Raumtemperatur gerührt. Anschließend wurde am Rotationsverdampfer ein Wassergehalt von 10 % eingestellt. Danach wurden 176 mg Zimtaldehyd (1,33 mmol) und 3 ml Diisopropylether (gesättigt mit 0,02 M Histidin-Puffer, pH 6,8) bei Raumtemperatur zugesetzt. Nach 10 min des Rührens bei Raumtemperatur wurden 200 µl wasserfreier Blausäure zugegeben und das Reaktionsgefäß druckdicht verschlossen. Das Reaktionsgemisch wurde 1 Stunde bei Raumtemperatur reagieren gelassen, anschließend wurde das immobilisierte Enzym abfiltriert, der Niederschlag dreimal mit je 10 ml Diisopropylether gewaschen und die organische Phase über wasserfreiem Natriumsulfat getrocknet. Abdestillieren des Lösungsmittels und anschließende säulenchromatographische Reinigung ergab 171 mg eines leicht gelblichen Feststoffes (81 % isolierte Ausbeute).
[α]_{D}²⁰-27,1° (c 1,85; CHCl₃).
2 Moläquivalente Imidazol (147 mg) und 1,5 Moläquivalente TBDMS-CI (243 mg) wurden unter Eiskühlung in 10 ml trockenem Dimethylformamid gelöst und 15 min bei Raumtemperatur gerührt. Anschließend wurde das gereinigte Cyanhydrin als Feststoff zugesetzt und 1 Stunde bei Raumtemperatur gerührt. Danach wurde mit 15 ml destilliertes Wasser verdünnt und die so erhaltene Lösung zweimal mit je 15 ml Diethylether extrahiert, mit wasserfreiem Natriumsulfat getrocknet und das Lösungsmittel unter vermindertem Druck entfernt. Nach Säulenchromatographie wurden 279 mg reines (S)-(-)-2-((tert-
Butyldimethylsilyl)oxy)-4-phenyl-(E)-but-3-ennitril (95 % isolierte Ausbeute) erhalten.
[α]_{D}²⁰-6,20° (c 0,55, CHCl₃).
e.e. = 92 % (gaschromatographisch bestimmt)
¹H-NMR: δ (ppm) 0,21 (s, 3H); 0,24 (s, 3H); 0,97 (s, 9H); 5,14 (d, 1H); 6,20 (dd, 1H); 6,83 (d, 1H); 7,33 - 7,44 (m, 5H).
¹³C-NMR: 135,17; 133,71; 128,83; 127,04; 123,77; 118,55; 62,73; 25,63; 18,26; -4,87; -4,92.

### Beispiel 5:

0,5 g Avicel-Cellulose wurden in 5 ml 0,01 M Natriumacetatpuffer (pH 5,4) suspendiert und 1,5 Stunden bei Raumtemperatur geschüttelt. Nach Filtration wurde der Niederschlag mit 2,7 ml Enzymlösung (390 lU/ml) aus Pichia Pastoris versetzt und 0,5 Stunden bei Raumtemperatur gerührt. Anschließend wurde am Rotationsverdampfer auf 30% Wassergehalt eingeengt und in 5 ml Diisopropylether suspendiert. Nach Zugabe von 230 µl 3-Phenoxybenzaldehyd (1,33 mmol) wurden 200 µl wasserfreie Blausäure (5,25 mmol) zugesetzt, das Reaktionsgefäß druckdicht verschlossen und 2 Stunden bei Raumtemperatur reagieren gelassen. Anschließend wurde mit 20 ml Diisopropylether verdünnt, 15 min bei Raumtemperatur gerührt, abfiltriert, die organische Phase mit wasserfreiem Natriumsulfat getrocknet und das Lösungsmittel im Vakuum entfernt. Säulenchromatographische Reinigung ergab 293 mg farbloses Öl (98 % isolierte Ausbeute).
[α]_{D}²⁰-27,9° (c 0,9, CHCl₃)
Das gereinigte Cyanhydrin wurde in 20 ml trockenem Methylenchlorid gelöst und mit 2 Moläquivalenten Acetanhydrid und 2 Moläquivalenten Pyridin bei Raumtemperatur über Nacht zur Reaktion gebracht. Anschließend wurde mit je 20 ml 5 % H₂SO₄, destilliertem Wasser und gesättigter Natriumhydrogencarbonat-Lösung gewaschen, mit wasserfreiem Natriumsulfat getrocknet und das Lösungsmittel bei vermindertem Druck abdestilliert. Säulenchromatographische Reinigung ergab 338 mg eines farblosen Öles (97 % isolierte Ausbeute).
[α]_{D}²⁰-7,10° (c 1,0, CHCl₃).
e.e. = 94 % (gaschromatographisch bestimmt)
¹H-NMR: δ (ppm) 2,18 (s, 3H); 6,36 (s, 1H); 7,01 - 7,44 (m, 9H).
¹³C-NMR: 169,00; 158, 52; 156,52; 133,80; 130,88; 130,24; 124,35; 122,33; 120,26; 119,64; 117,96; 116,12; 62,69; 20,66.

### Beispiel 6:

1,33 mmol Cyclohexancarbaldehyd (161 µl) wurden in 2,7 ml Diisopropylether gelöst und auf 0 - 5°C gekühlt. 1,1 ml wässrige Enzymlösung (900 lU/ml) und 200 µl wasserfreie Blausäure wurden zugesetzt, das Reaktionsgefäß druckdicht verschlossen und 1 Stunde bei 0 - 5°C gerührt. Anschließend wurde dreimal mit je 5 ml Diisopropylether extrahiert, mit wasserfreiem Natriumsulfat getrocknet und das Lösungsmittel unter vermindertem Druck abdestilliert. Nach Säulenchromatographie wurden 180 mg eines farblosen Öles (97 % isolierte Ausbeute) erhalten.
[α]_{D}²⁰-9,80° (c 1,25, CHCl₃)
Lösen in 15 ml Methylenchlorid, Zugabe von 2 Moläquivalenten Acetanhydrid, 2 Moläquivalenten Pyridin, Reaktion über Nacht bei Raumtemperatur und anschließender Extraktion mit je 20 ml 5 % H₂SO₄, destilliertem Wasser und gesättigter Natriumhydrogencarbonat-Lösung, trocknen mit wasserfreiem Natriumsulfat, abdestillieren des Lösungsmittels unter vermindertem Druck und anschließender säulenchromatographische Reinigung ergab 230 mg eines farblosen, dünnflüssigen Öles (98 % isolierte Ausbeute).
[α]_{D}²⁰-64,8° (c 0,80 CH₂Cl₂)
e.e.>99 % (gaschromatographisch bestimmt)
¹H-NMR: δ(ppm) 1,11 - 1,30 (m, 5H); 1,69 - 1,91 (m, 6H); 2,13 (s, 3H); 5,16 (d, 1H).
¹³C-NMR: 169,35; 116,35; 65,77; 40,21; 28,30; 28,08; 25,94; 25,54; 25,47; 20,49.

### Beispiel 7:

0,5 g mikrokristalline Cellulose wurde in 2,7 ml 0,02 M Histidin-Puffer bei Raumtemperatur 1 Stunde quellengelassen. Anschließend wurde das Quellmittel durch Filtration entfernt und der Träger mit 1,1 ml wässriger Enzymlösung (900 lU/ml) versetzt. Danach wurde der Niederschlag am Rotationsverdampfer auf einen Wassergehalt von 25 % eingestellt. Der Rückstand wurde in 3 ml Diisopropylether (gesättigt mit 0,02 M Histidin-Puffer) suspendiert und 1,33 mmol Furfural (220 µl) zugesetzt. Nach zehnminütigem Rühren bei Raumtemperatur wurden 5,25 mmol wasserfreie Blausäure (200 µl) zugegeben, der Kolben druckdicht verschlossen und die Reaktionsmischung 1 Stunde bei Raumtemperatur gerührt. Danach wurde das Enzymimmobilisat abfiltriert und zweimal mit je 10 ml Diisopropylether nachgewaschen. Die vereinigten organischen Phasen wurden über wasserfreiem Natriumsulfat getrocknet und das Lösungsmittel unter vermindertem Druck entfernt. Säulenchromatographische Reinigung ergab das reine Cyanhydrin als farbloses Öl (160 mg, 98 % isolierte Ausbeute).
[α]_{D}²⁰-26,1 ° (c 1.2, CHCl₃)
Das derart gereinigte Cyanhydrin wurde in 15 ml Diethylether gelöst und mit 2 Moläquivalenten Acetanhydrid und mit 2 Moläquivalenten Pyridin versetzt. Nach 10 Stunden Reaktionszeit wurde mit je 20 ml 5 % H₂SO₄, destilliertem Wasser und gesättigter Natriumhydrogencarbonat-Lösung gewaschen, mit wasserfreiem Natriumsulfat getrocknet und nach Filtration die Lösung unter vermindertem Druck eingeengt. Nach Säulenchromatographie wurden 210 mg eines farblosen Öles (99 % isolierte Ausbeute) erhalten.
[α]_{D}²⁰-23,3° (c 1,4, CHCl₃)
e.e. = 94 % (gaschromatographisch bestimmt)
¹H-NMR: δ (ppm) 2,17 (s, 3H); 6,45 (dd, 1H); 6,47 (s, 1H); 6,68 (dd, 1H); 7,51 (dt, 1H).
¹³C-NMR: 168,68; 145,10; 144,03; 112,64; 111,18; 114,17; 55,77; 20,38.

### Beispiel 8:

Analog Beispiel 7 wurde mit Furan-3-carbaldehyd nach säulenchromatographischer Reinigung 157 mg (-)-2-(3-Furyl)-2-hydroxyacetonitril (96 % isolierte Ausbeute) erhalten
[α]_{D}²⁰-14,6° (c 1,85, CHCl₃)
Nach Acetylierung und entsprechender Aufarbeitung und säulenchromatographischer Reinigung wurden 208 mg (-)-2-Acetoxy-2-(3-furyl)acetonitril (99 % isolierte Ausbeute) erhalten.
[α]_{D}²⁰-1 11,4° (c 2, CHCl₃)
e.e. = 97 % (gaschromatographisch bestimmt)
¹H-NMR: δ (ppm) 2,16 (s, 3H); 6,36 (s, 1H); 6,54 (dd, 1H); 7,47 (dd, 1H); 7,68 (m, 1H).
¹³C-NMR: 169,15; 144,77; 142,75; 118,34; 109,46; 115,86; 55,78; 20,63.

### Beispiel 9:

1,33 mmol Hexanal (133 mg) wurden in 3 ml wassergesättigtem Diisopropylether gelöst. Bei Raumtempertur wurde 1,1 ml wässriger Hydroxynitril Lyase-Lösung (900 IU/ml) zugesetzt und 5 min bei Raumtemperatur gerührt. Danach wurden 300 µl wasserfreie Blausäure (7,88 mmol) zugegeben und das Reaktionsgefäß druckdicht verschlossen. Nach 1,5 Stunden des Rührens bei Raumtemperatur wurden die Phasen getrennt, die wässrige Phase dreimal mit je 5 ml Diisopropylether extrahiert und die vereinigten Etherphase mit wasserfreiem Natriumsulfat getrocknet. Das Lösungsmittel wurde unter vermindertem Druck entfernt und das derart erhaltene rohe Cyanhydrin einer säurelchromatographischen Reinigung unterworfen. Es wurden 140 mg (S)-2-Hydroxyheptannitril (83 % isolierte Ausbeute) erhalten.
[α]_{D}²⁰-14,1° (c 1,5, CHCl₃)
Das gereinigte Cyanhydrin wurde in 5 ml trockenem Diethylether gelöst, mit 2 Moläquivalenten Acetanhydrid und 2 Moläquivalenten Pyridin versetzt und 8 Stunden bei Raumtemperatur gerührt. Anschließend wurde mit je 10 ml 5 % H₂SO₄, destilliertem Wasser und gesättigter NaHCO₃-Lösung extrahiert, über wasserfreiem Natriumsulfat getrocknet und das Lösungsmittel unter vermindertem Druck entfernt. Nach säulenchromatographischer Reinigung wurden 182 mg (S)-2-Acetoxyheptannitril (98 % isolierte Ausbeute) erhalten.
e.e. = 96 % (gaschromatographisch bestimmt).
¹H-NMR: δ(ppm) 0,90 (t, 3H); 1,21 - 1,60 (m, 6H); 1,90 (q, 2H); 2,15 (s, 3H); 5,32 (t, 1H).
¹³C-NMR: 169,1; 116,9; 61,10; 32, 11; 30,89;24,10; 22,23; 20,29; 13,76.

### Beispiel 10:

2 mmol 2-Pentanon (21µl) wurden in 10 ml Diisopropylether gelöst und unter Rühren auf 0 - 5°C abgekühlt. Anschließend wurden bei 0 - 5 °C 4 mmol Kaliumcyanid (260 mg) mit 0,1 M Zitronensäure versetzt, sodaß ein pH-Wert von 3,5 erreicht wurde. Diese wässrige Lösung wurde zur organischen Phase zugegeben. Nach Zusatz von 1000 IU Hnl (1 ml Enzymlösung (1000 IU/ml)) wurde das Reaktionsgefäß druckdicht verschlossen und 1,5 Stunden bei 0 - 5°C gerührt. Danach wurde mit 10 ml Diisopropylether verdünnt und damit das Reaktionsgemisch extrahiert, die wässrige Phase abgetrennt und ein zweites Mal mit 20 ml Diisopropylether extrahiert. Die vereinigten organischen Phasen wurde über wasserfreiem Natriumsulfat getrocknet und unter vermindertem Druck zur Trockene eingeengt. Es wurden 136 mg (60 %) eines leicht gelblichen Öles erhalten. Nach Säulenchromatographie wurde das reine Cyanhydrin in 38 % Ausbeute (86 mg) als farbloses Öl erhalten.
[α]_{D}²⁰-2,5° (c 1,46; CHCl₃)
Das gereinigte Cyanhydrin wurde in 2 ml absolutem Methylenchlorid gelöst und bei Raumtemperatur mit 2 Moläquivalenten Acetanhydrid und mit 2 Moläquivalenten Pyridin versetzt und unter Feuchtigkeitsauschluß 34 Stunden bei Raumtemperatur gerührt. Anschließend wurden 350 µl absolutes Methanol zugesetzt und 0,5 Stunden bei Raumtemperatur gerührt. Danach wurden mit 10 ml Methylenchlorid verdünnt und mit je 10 ml 5% H₂SO₄ destilliertem Wasser und gesättigter NaHCO₃-Lösung extrahiert und über wasserfreiem Natriumsulfat getrocknet und das Lösungsmittel unter vermindertem Druck entfernt. Es wurden 112 mg (95 %) (S)-2-Acetoxy-2-methylpentannitril erhalten.
e.e. = 75 % (gaschromatographisch bestimmt)
[α]_{D}²⁰ = -8,5° (c 1,94; CHCl₃)
¹H-NMR: δ (ppm) 0,99 (t, 3H); 1,41 - 1,70 (m, 2H); 1,73 (s, 3H); 1,77 - 2,04 (m, 2H); 2,09 (s, 3H);
¹³C-NMR: 168,88; 118,83; 72,00; 41,79; 24,60; 21,17; 17,33; 13,75.

### Beispiel 11:

500 µl 3-Methyl-2-pentanon (4mmol) wurden in 15 ml Diisopropylether unter Rühren gelöst, auf 0 - 5 °C abgekühlt und eine wässrige Lösung von 520 mg Kaliumcyanid in 20 ml 0,5 M Zitronensäure zugesetzt. Nach Zugabe von 2 ml Enzymlösung (1000 lU/ml) wird das Reaktionsgefäß druckdicht verschlossen und 5 Stunden bei 0 - 5°C gerührt. Anschließend wird mit 30 ml Diisopropylether verdünnt und die Reaktionsmischung extrahiert. Nach Abtrennung der organischen Phase wurde die wässrige Phase ein zweites Mal mit 30 ml Diisopropylether ausgeschüttelt. Die wässrige Phase wurde verworfen, die vereinigten organischen Phasen über wasserfreiem Natriumsulfat getrocknet und das Lösungsmittel unter vermindertem Druck abdestilliert. Nach Säulenchromatographie wurden 193 mg (S)-2-Hydroxy-2,4-dimethylpentannitril (38 %) erhalten.

Derivatisierung und anschließende Aufarbeitung nach der in Beispiel 10 beschriebenen Art und Weise ergaben 231 mg (S)-2-Acetoxy-2,4-dimethylpentannitril (90 %) als farbloses Öl.
[α]_{D}²⁰-28,5° (c 1,75; CHCl₃)
e.e. = 96 % (gaschromatographisch bestimmt)
¹H-NMR: δ (ppm) 1,01; 1,04 (2d, 6H); 1,43 (s, 2H); 1,75 (s, 3H); 1,83 - 2,04 (m, 1H); 2,09 (s, 3H)
¹³C-NMR: 168, 87; 119,04; 71,69; 30,36; 25,22; 24,84; 23,68; 23,52; 21,29.

### Beispiel 12:

10 mmol Pinakolon (1,25 g) wurden in 30 ml Methyl-t-butylether gelöst, mit 5 ml Enzymlösung (1 000 lU/ml) versetzt und unter Rühren auf 0 - 5°C abgekühlt. Parallel dazu wurden unter Eiskühlung 20 mmol Kaliumcyanid in 250 ml 0,1 M wässriger Zitronensäure gelöst. Die derart hergestellte Blausäurelösung wurde dem Reaktionsgemisch zugesetzt und das Reaktionsgefäß druckdicht verschlossen. Nach einer Reaktionszeit von 2,5 h wurde mit 50 ml Methyl-t-butylether verdünnt und die Reaktionslösung extrahiert. Die wässrige Phase wurde abgetrennt, nochmals mit 80 ml Methyl-t-butylether ausgeschüttelt und anschließend verworfen. Die vereinigten organischen Phasen wurden mit wasserfreiem Natriumsulfat getrocknet und das Lösungsmittel unter vermindertem Druck entfernt. Nach Säulenchromatographie wurden 0,95 g (S)-2-Hydroxy-2,3,3-trimethylbutannitril (75 %) als weißen Feststoff (m.p. 98 - 99°C) erhalten.
Anschließend wurde das Rohprodukt in 10 ml wasserfreiem Diethylether gelöst und unter Eiskühlung je 2,5 Moläquivalente Acetylchlorid und Pyridin zugesetzt.
Nach Zugabe von 30 mg 4-N,N-Dimethylaminopyridin wurde 48 Stunden bei Raumtemperatur reagieren gelassen. Anschließend wurden 3 Moläquivalente trockenes Methanol zugesetzt und weitere 30 min bei Raumtemperatur gerührt. Schießlich wurde diese Lösung mit 20 ml Diethylether verdünnt und mit je 30 ml verdünnter, wässrige Schwefelsäure, destilliertem Wasser und gesättigter, wässriger Natriumhydrogencarbonatlösung extrahiert. Nach Trocknung mit wasserfreiem Natriumsulfat wurde das Lösungsmittel unter vermindertem Druck entfernt. Nach Säulenchromatographie wurden 1,21 g (S)-2-Acetoxy-2,3,3-trimethylbutannitril (96 %) als farbloses Öl erhalten.
[α]_{D}²⁰-40,5 (c 2,25, CHCl₃)
e.e. =78 % (gaschromatographisch bestimmt)
¹H-NMR: 1,11 (s, 9H); 1,69 (s, 3H); 2,09 (s, 3H).
¹³C-NMR: 168,97; 118,10; 78,07; 38,39; 24,66; 21,20; 18,93.

### Beispiel 13:

Einmalige Verwendung von immobilisierter (S)-Hnl aus Pichia pastoris in Ethylacetat

2 g Avicel wurden in 13,4 ml 0,02 M Natriumacetat-Lösung (pH 5,4) 1 Stunde lang gequollen. Danach wurde filtriert und der Rückstand mit 13,4 ml Enzymlösung (390 ID/ml) 5 Minuten lang behandelt. Wasser wurde schonend bei 40°C und 10 - 20 mbar abdestilliert. Der Wassergehalt vom Immobilisat betrug nach dieser Behandlung 40 %.
Es wurden 13,5 ml Ethylacetat, welches vorher mit 0,01 M Natriumacetat-Lösung gesättigt wurde, vorgelegt. Dazu wurde das Immobilisat eingerührt. Bei 20°C wurden 0,71 g frisch destillierter Benzaldehyd (6,6 mmol) und 1,00 ml Blausäure (25,5 mmol) zugegeben. Der Reaktionsverlauf wurde durch Abnahme der Konzentration von Benzaldehyd photometrisch verfolgt.
Nach 1 Stunde wurde ein Reaktionsumsatz von 95 % erreicht. Die Reaktionslösung wurde filtriert und mehrmals mit 10 ml Ethylacetat nachgewaschen. Das Filtrag wurde unter Vakuum eingeengt. Als Rückstand verblieben 0,8 g S-Mandelsäurenitril (90 % chemische Ausbeute) mit einem Enantiomerenüberschluß von 98 %.

### Beispiel 14

Mehrmalige Verwendung von immobilisierter (S)-Hnl aus Pichia pastoris in Ethylacetat

1 g Avicel wurden in 6,7 ml 0,02 M Natriumacetat-Lösung (pH 5,4) 1 Stunde lang gequollen. Danach wurde filtriert und der Rückstand mit 1,68 ml Enzymlösung (330 lU/ml) 5 Minuten lang behandelt. Wasser wurde schonend bei 40°C und 10 - 20 mbar abdestilliert. Der Wassergehalt vom Immobilisat betrug nach dieser Behandlung 40 %.
Es wurden 13,5 ml Ethylacetat, welches vorher mit 0,01 M Natriumacetat-Lösung gesättigt wurde, vorgelegt. Dazu wurde das Immobilisat eingerührt. Bei 20°C wurden 0,71 g frisch destillierter Benzaldehyd (8,8 mmol) und 1,0 ml Blausäure (25,5 mmol) zugegeben. Der Reaktionsverlauf wurde durch Abnahme der Konzentration von Benzaldehyd photometrisch verfolgt.
Nach 45 Minuten wurde ein Reaktionsumsatz von 90 % erreicht. Die Reaktionslösung wurde filtriert und mehrmals mit 10 ml Ethylacetat nachgewaschen. Das Filtrat wurde unter Vakuum eingeengt. Als Rückstand verblieben 0,86 g S-Mandelsäurenitril mit einem Enantiomerenüberschuß von 98,4.
Das abfiltrierte Immobilisat wurde in weiteren Ansätzen mit denselben Einwaagen wie bereits oben beschrieben eingerührt und in gleicher Weise aufgearbeitet.

### Ergebnisse:

| | g (S)-Mandelsäurenitril | % d. Theorie | % ee |
|---|---|---|---|
| 1. Verwendung | 0,86 | 97 | 98,4 |
| 2. Verwendung | 0,74 | 83 | 97,2 |
| 3. Verwendung | 0,76 | 85 | 94,1 |

### Beispiel 15:

Mehrmalige Verwendung von immobilisierter (S)-Hnl aus Pichia pastoris in Methyl tert. butylether (MTBE)

2 g Avicel wurden in 13,4 ml 0,02 M Natriumacetat-Lösung (pH 5,4) 1 Stunde lang gequollen. Danach wurde filtriert und der Rückstand mit 3,35 ml Enzymlösung (330 IU/ml) 5 Minuten lang behandelt. Wasser wurde schonend bei 40°C und 10 - 20 mbar abdestilliert. Der Wassergehalt vom Immobilisat betrug nach dieser Behandlung 40 %.
Es wurden 13,5 ml MTBE, welches vorher mit 0,01 M Natriumacetat-Lösung gesättigt wurde, vorgelegt. Dazu wurde das Immobilisat eingerührt. Bei 20°C wurden 0,71 g frisch destillierter Benzaldehyd (6,6 mmol) und 1,00 ml Blausäure (25,5 mmol) zugegeben. Der Reaktionsverlauf wurde durch Abnahme der Konzentration von Benzaldehyd photometrisch verfolgt. Nach 45 Minuten betrug der Umsatz 90 %.
Das abfiltrierte Immobilisat wurde in weiteren Ansätzen mit denselben Einwaagen wie bereits oben beschrieben eingerührt und in gleicher Weise aufgearbeitet.

### Ergebnisse:

| | g (S)-Mandelsäurenitril | % d. Theorie | % ee |
|---|---|---|---|
| 1. Verwendung | 0,75 | 84 | 98,9 |
| 2. Verwendung | 0,77 | 86 | 98,0 |
| 3. Verwendung | 0,73 | 82 | 96,8 |
| 4. Verwendung | 0,71 | 80 | 88,3 |

### Beispiel 16:

Mehrmalige Verwendung von immobilisierter (S)-Hnl aus Pichia pastoris in Diisopropylether (DIPE)

2 g Avicel wurden in 13,4 ml 0,02 M Natriumacetat-Lösung (pH 5,4) 1 Stunde lang gequollen. Danach wurde filtriert und der Rückstand mit 3,35 ml Enzymlösung (330 IU/ml) 5 Minuten lang behandelt. Wasser wurde schonend bei 40°C und 10 - 20 mbar abdestilliert. Der Wassergehalt vom Immobilisat betrug nach dieser Behandlung 40 %.
Es wurden 13,5 ml DIPE, welches vorher mit 0,01 M Natriumacetat-Lösung gesättigt wurde, vorgelegt. Dazu wurde das Immobilisat eingerührt. Bei 20°C wurden 0,71 g frisch destillierter Benzaldehyd (6,6 mmol) und 1,00 ml Blausäure (25,5 mmol) zugegeben. Der Reaktionsverlauf wurde durch Abnahme der Konzentration von Benzaldehyd photometrisch verfolgt. Nach 1 Stunde betrug der Umsatz mehr als 90 %.
Das abfiltrierte Immobilisat wurde in weiteren Ansätzen mit denselben Einwaagen wie bereits oben beschrieben eingerührt und in gleicher Weise aufgearbeitet.

### Ergebnisse:

| | g (S)-Mandelsäurenitril | % d. Theorie | % ee |
|---|---|---|---|
| 1. Verwendung | 0,74 | 83 | 97,9 |
| 2. Verwendung | 0,72 | 81 | 97,7 |
| 3. Verwendung | 0,72 | 82 | 95,0 |
| 4. Verwendung | 0,75 | 84 | 96,8 |
| 5. Verwendung | 0,77 | 86 | 95,4 |
| 6. Verwendung | 0,77 | 86 | 94,7 |
| 7. Verwendung | 0,75 | 84 | 92,5 |

### Beispiel 17

Einmalige Verwendung von minimaler Menge immobilisierter (S)-Hnl aus Pichia pastoris in Diisopropylether (DIPE)

1 g Avicel wurden in 6,7 ml 0,07 M Natriumacetat-Lösung (pH 5,4) 1 Stunde lang gequollen. Danach wurde filtriert und der Rückstand mit 0,8 ml Enzymlösung (194 lU/ml) 5 Minuten lang behandelt. Wasser wurde schonend bei 40°C und 10 - 20 mbar abdestilliert. Der Wassergehalt vom Immobilisat betrug nach dieser Behandlung 40 %.
Es wurden 13,5 ml DIPE, welches vorher mit 0,01 M Natriumacetat-Lösung gesättigt wurde, vorgelegt. Dazu wurde das Immobilisat eingerührt. Bei 20°C wurden 0,71 g frisch destillierter Benzaldehyd (6,6 mmol) und 1,0 ml Blausäure (25,5 mmol) zugegeben. Der Reaktionsverlauf wurde durch Abnahme der Konzentration von Benzaldehyd photometrisch verfolgt. Nach 2 Stunden betrug der Umsatz 86 %.

### Ergebnisse:

| | g (S)-Mandelsäurenitril | % d. Theorie | % ee |
|---|---|---|---|
| 1. Verwendung | 0,86 | 97 | 96,5 |

### Beispiel 18:

Einmalige Verwendung von immobilisierter (S)-Hnl aus Pichia pastoris in Ethylacetat und Acetoncyanhydrin als Cyanid-Donor

2 g Avicel wurden in 13,5 ml 0,02 M Natriumacetat-Lösung (pH 5,4) 1,6 Stunden lang gequollen. Danach wurde filtriert und der Rückstand mit 3,35 ml Enzymlösung (390 IU/ml) 5 Minuten lang behandelt. Wasser wurde schonend bei 40°C und 10 - 20 mbar abdestilliert. Der Wassergehalt vom Immobilisat betrug nach dieser Behandlung 40 %.
Es wurden 13,5 ml Ethylacetat, welches vorher mit 0,01 M Natriumacetat-Lösung gesättigt wurde, vorgelegt. Dazu wurde das Immobilisat eingerührt. Bei 20°C wurden 0,71 g frisch destillierter Benzaldehyd (6,6 mmol) und 3,3 ml frisch destilliertes Acetoncyanhydrin (33,8 mmol) zugegeben. Der Reaktionsverlauf wurde durch Abnahme der Konzentration von Benzaldehyd photometrisch verfolgt. Nach 4 Stunden betrug der Umsatz 78 %.
Die Reaktionslösung wurde filtriert und mehrmals mit 10 ml Ethylacetat nachgewaschen. Das Filtrat wurde unter Vakuum eingeengt. Der Rückstand enthielt neben nicht umgesetztem Acetoncyanhydrin das gewünschte (S)-Mandelsäurenitril mit einem Enantiomerenüberschuß von 95,0 %.

### Beispiel 19:

Mehrmalige Verwendung von nicht-immobilisierter (S)-Hnl aus Pichia pastoris in Diisopropylether (DIPE) in einem Zweiphasengemisch.

Es wurden 13,5 ml DIPE, welches vorher mit 0,01 M Natriumacetat-Lösung gesättigt wurde, vorgelegt. Dazu wurden 1,6 ml Enzymlösung (194 lU/ml) eingerührt. Bei 20°C wurden 0,71 g frisch destillierter Benzaldehyd (6,6 mmol) und 1,0 ml Blausäure (25,5 mmol) zugegeben. Der Reaktionsverlauf wurde durch Abnahme der Konzentration von Benzaldehyd photometrisch verfolgt.
Die Phasen der Reaktionslösung wurden getrennt. Die wässrige Phase wurde mehrmals mit DIPE behandelt. Die so erhaltenen DIPE-Lösungen wurden vereinigt und der Reaktionslösung zugegeben. Das Lösungsmittel wurde bei 40°C und 20 mbar abdestilliert. Als Rückstand verblieb das S-Mandelsäurenitril.
Die wässrige Enzymlösung wurde in den nächsten Ansätzen mit den gleichen Einwaagen eingesetzt.

### Ergebnisse:

| | Std/Umsatz | g (S)-Mandelsäurenitril | % d. Theorie | % ee |
|---|---|---|---|---|
| 1. Verwendung | 1,0/93 | 0,80 | 90 | 99,1 |
| 2. Verwendung | 1,5/86 | 0,81 | 91 | 98,7 |
| 3. Verwendung | 2,0/90 | 0,71 | 80 | 99,0 |
| 4. Verwendung | 3,5/79 | 0,79 | 89 | 97,5 |

### Beispiel 20:

Einmalige Verwendung von minimaler Menge nicht-immobilisierter (S)-Hnl aus Pichia pastoris in Diisopropylether (DIPE) in einem Zweiphasen-Gemisch

Es wurden 13,5 ml DIPE, welches vorher mit 0,01 M Natriumacetat-Lösung gesättigt wurde, vorgelegt. Dazu wurden 0,8 ml Enzymlösung (194 lU/ml) eingerührt. Bei 20°C wurden 0,71 g frisch destillierter Benzaldehyd (6,6 mmol) und 1,0 ml Blausäure (25,5 mmol) zugegeben. Der Reaktionsverlauf wurde durch Abnahme der Konzentration von Benzaldehyd photometrisch verfolgt.
Die Phasen der Reaktionslösung wurden getrennt. Die wässrige Phase wurde mehrmals mit DIPE behandelt. Die so erhaltene DIPE-Lösungen wurden vereinigt und der Reaktionslösung zugegeben. Das Lösungsmittel wurde bei 40°C und 20 mbar abdestilliert. Als Rückstand verblieb das (S)-Mandelsäurenitril

### Ergebnisse:

| | Std/Umsatz | g (S)-Mandelsäurenitril | % d. Theorie | % ee |
|---|---|---|---|---|
| 1. Verwendung | 2,0/86 | 0,86 | 97 | 99,1 |

### Beispiel 21:

Mehrmalige Verwendung von nicht-immobilisierter (S)-Hnl aus Pichia pastoris in Methyl tert.Butylether in einem Zweiphasen-Gemisch

Es wurden 13,5 ml MTBE, welches vorher mit 0,01 M Natriumacetat-Lösung gesättigt wurde, vorgelegt. Dazu wurden 5,5 ml Enzymlösung (194 lU/ml) eingerührt. Bei 20°C wurden 0,71 g frisch destillierter Benzaldehyd (6,6 mmol) und 1,0 ml Blausäure (25,5 mmol) zugegeben. Der Reaktionsverlauf wurde durch Abnahme der Konzentration von Benzaldehyd photometrisch verfolgt.
Die Phasen der Reaktionslösung wurden getrennt. Es erfolgte keine Isolierung von (S)-Mandelsäurenitril, es wurde nur die Aktivität der Enzymlösung gemessen.

Die wässrige Enzymlösung wurde in den nächsten Ansätzen mit den gleichen Einwaagen eingesetzt.

### Ergebnisse:

| | Std/Umsatz | Aktivität (ID/ml) |
|---|---|---|
| 1. Verwendung | 1/92,5% | 194 |
| 2. Verwendung | 0,75/93,6% | 119 |
| 3. Verwendung | 0,75/87,2 | 81,5 |
| 4. Verwendung | 1,5/87,4 | 61,4 |

### Beispiel 22:

Einmalige Verwendung von minimaler Menge nicht-immobilisierter (S)-Hnl aus Pichia pastoris in Methyl tert. butylether (MTBE) in einem Zweiphasen-Gemisch

Es wurden 13,5 ml MTBE, welches vorher mit 0,01 M Natriumacetat-Lösung gesättigt wurde, vorgelegt. Dazu wurden 0,8 ml Enzymlösung (194 lU/ml) eingerührt. Bei 20°C wurden 0,71 g frisch destillierter Benzaldehyd (6,6 mmol) und 1,0 ml Blausäure (25,5 mmol) zugegeben. Der Reaktionsverlauf wurde durch Abnahme der Konzentration von Benzaldehyd photometrisch verfolgt.
Die Phasen der Reaktionslösung wurden getrennt. Die wässrige Phase wurde mehrmals mit MTBE behandelt. Die so erhaltene MTBE-Lösungen wurden vereinigt und der Reaktionslösung zugegeben. Das Lösungsmittel wurde bei 40°C und 20 mbar abdestilliert. Als Rückstand verblieb das (S)-Mandelsäurenitril.

### Ergebnisse:

| | Std/Umsatz | g (S)-Mandelsäurenitril | % d. Theorie | % ee |
|---|---|---|---|---|
| 1. Verwendung | 2,0/86 | 0,85 | 96 | 99,0 |

### Beispiel 23:

Einmalige Verwendung von minimaler Menge nicht-immobilisierter (S)-Hnl aus Pichia pastoris in Methyl tert.butylether (MTBE) in einem Zweiphasen-Gemisch mit unterschiedlichen Waservolumina.

Es wurden 13,5 ml MTBE, welches vorher mit 0,01 M Natriumacetat-Lösung gesättigt wurde, vorgelegt. Dazu wurden 0,8 ml Enzymlösung (194 lU/ml) mit unterschiedlichen Mengen an Wasser eingerührt. Bei 20°C wurden 0,71 g frisch destillierter Benzaldehyd (6,6 mmol) und 1,0 ml Blausäure (25,5 mmol) zugegeben. Der Reaktionsverlauf wurde durch Abnahme der Konzentragion von Benzaldehyd photometrisch verfolgt.
Die Phasen der Reaktionslösung wurden getrennt. Die wässrige Phase wurde mehrmals mit MTBE behandelt. Die so erhaltene MTBE-Lösungen wurden vereinigt und der Reaktionslösung zugegeben. Das Lösungsmittel wurde bei 40°C und 20 mbar abdestilliert. Als Rückstand verblieb das (S)-Mandelsäurenitril.

### Ergebnisse:

| Wasser-Zusatz | Std/Umsatz | g (S)-Mandelsäurenitril | % d. Theorie | % ee |
|---|---|---|---|---|
| 1,2 ml | 2,0/86 | 0,88 | 99 | 98,3 |
| 3,2 ml | 1,0/90 | 0,80 | 90 | 98,4 |
| 9,2 ml | 1,0/89 | 0,86 | 97 | 97,5 |

### Beispiel 24:

Einmalige Verwendung von minimaler Menge nicht-immobilisierter (S)-Hnl aus Pichia pastoris in Methyl tert. butylether (MTBE) in einem Zweiphasen-Gemisch bei unterschiedlichen pH-Werten.

Es wurden 13,5 ml MTBE, welches vorher mit 0,01 M Natriumacetat-Lösung gesättigt wurde, vorgelegt. Dazu wurden 0,8 ml Enzymlösung (194 ID/ml) und 9,2 ml Wasser eingerührt. Vor der Zugabe wurde der pH-Wert mit Phosphorsäure eingestellt. Bei 20°C wurden 0,71 g frisch destillierter Benzaldehyd (6,6 mmol) und 1,0 ml Blausäure (25,5 mmol) zugegeben. Der Reaktionsverlauf wurde durch Abnahme der Konzentration von Benzaldehyd photometrisch verfolgt.
Die Phasen der Reaktionslösung wurden getrennt. Die wässrige Phase wurde mehrmals mit MTBE behandelt. Die so erhaltene MTBE-Lösungen wurden vereinigt und der Reaktionslösung zugegeben. Das Lösungsmittel wurde bei 40°C und 20 mbar abdestilliert. Als Rückstand verblieb das (S)-Mandelsäurenitril.

### Ergebnisse:

| pH-Wert | Std/Umsatz | g (S)-Mandelsäurenitril | % d. Theorie | % ee |
|---|---|---|---|---|
| 5,7 | 1,0/89 | 0,88 | 97 | 97,5 |
| 5,0 | 1,0/90 | 0,85 | 0,5 | 98,8 |
| 4,0 | 22,0/67 | keine Isolierung | | |
| 3,0 | 22,0/11 | keine Isolierung | | |

### Beispiel 25

Einmalige Verwendung von minimaler Menge nicht-immobilisierter (S)-Hnl aus Pichia pastoris in Methyl tert. butylether (MTBE) in einem Zweiphasen-Gemisch bei verminderter Lösungsmittelmenge

Es wurden 6,7 ml MTBE, welches vorher mit 0,01 M Natriumacetat-Lösung gesättigt wurde, vorgelegt. Dazu wurden 0,8 ml Enzymlösung (194 IU/ml) eingerührt. Bei 20°C wurden 0,71 g frisch destillierter Benzaldehyd (6,6 mmol) und 1,0 ml Blausäure (25,5 mmol) zugegeben. Der Reaktionsverlauf wurde durch Abnahme der Konzentration von Benzaldehyd photometrisch verfolgt.
Die Phasen der Reaktionslösung wurden getrennt. Die wässrige Phase wurde mehrmals mit MTBE behandelt. Die so erhaltene MTBE-Lösungen wurden vereinigt und der Reaktionslösung zugegeben. Das Lösungsmittel wurde bei 40°C und 20 mbar abdestilliert. Als Rückstand verblieb das (S)-Mandelsäurenitril.

### Ergebnisse:

| | Std/Umsatz | g (S)-Mandelsäurenitril | % d. Theorie | % ee |
|---|---|---|---|---|
| 1. Verwendung | 2,0/92 | 0,88 | 99 | 94,4 |

### Beispiel 26:

Mehrmalige Verwendung von nicht-immobilisierter (S)-Hnl aus Pichia pastoris in Methyl tert. butylether (MTBE) in einem Zweiphasen-Gemisch unter Zusatz eines Stabilisator

Es wurden 13,5 ml MTBE, welches vorher mit 0,01 M Natriumacetat-Lösung gesättigt wurde, vorgelegt. Dazu wurden 5,5 ml Enzymlösung (151 lU/ml) und 55 mg einer nicht aktiven Hnl-Präparation aus Blättern von Hevea brasiliensis eingerührt. Bei 20°C wurden 0,71 g frisch destillierter Benzaldehyd (6,6 mmol) und 1,0 ml Blausäure (25,5 mmol) zugegeben. Der Reaktionsverlauf wurde durch Abnahme der Konzentration von Benzaldehyd photometrisch verfolgt.
Die Phasen der Reaktionslösung wurden getrennt. Die wässrige Phase wurde mehrmals mit MTBE behandelt. Die so erhaltene MTBE-Lösungen wurden vereinigt und der Reaktionslösung zugegeben. Das Lösungsmittel wurde bei 40°C und 20 mbar abdestilliert. Als Rückstand verblieb das (S)-Mandelsäurenitril.
Die wässrige Enzymlösung wurde in den nächsten Ansätzen mit den gleichen Einwaagen eingesetzt. Bei der 10. und 11. Verbindung wurden 0,5 ml Wasser zugesetzt.

### Ergebnisse:

| Verwendung | Std/Umsatz | g (S)-Mandelsäurenitril | % d. Theorie | % ee |
|---|---|---|---|---|
| 1. | 1/91 | 0,78 | 88 | 97,6 |
| 2. | 1/89 | 0,60 | 67 | 97,8 |
| 3. | 1/92 | 0,87 | 98 | 97,2 |
| 4. | 1/93 | 0,87 | 98 | 98,2 |
| 5. | 1/97 | 0,86 | 97 | 97,1 |
| 6. | 1/96 | 0,88 | 99 | 98,6 |
| 7. | 1/90 | 0,88 | 99 | 99,1 |
| 8. | 1/94 | 0,88 | 99 | 98,4 |
| 9. | 2/86 | 0,89 | 100 | 97,8 |
| 10. | 1/93 | 0,85 | 96 | 98,4 |
| 11. | 2,/93 | 0,83 | 93 | 98,9 |

### Beispiel 27:

Mehrmalige Verwendung von nicht-immobilisierter (S)-Hnl in aufgeschlossenen Zellen von Pichia pastoris ("Homogenat") in Diisopropylether (DIPE) in einem Zweiphasen-Gemsich

Es wurden 13,5 ml DIPE, welches vorher mit 0,01 M Natriumacetat-Lösung gesättigt wurde, vorgelegt. Dazu wurden 3,2 ml Homogenat (310 lU/ml) eingerührt. Bei 20°C wurden 0,71 g frisch destillierter Benzaldehyd (6,6 mmol) und 1,0 ml Blausäure (25,5 mmol) zugegeben. Der Reaktionsverlauf wurde durch Abnahme der Konzentration von Benzaldehyd photometrisch verfolgt.
Die Phasen der Reaktionslösung wurden getrennt. Die wässrige Phase wurde mehrmals mit DIPE behandelt. Die so erhaltene DIPE-Lösungen wurden vereinigt und der Reaktionslösung zugegeben. Das Lösungsmittel wurde bei 40°C und 20 mbar abdestilliert. Als Rückstand verblieb das (S)-Mandelsäurenitril.
Die wässrige Enzymlösung wurde in den nächsten Ansätzen mit den gleichen Einwaagen eingesetzt. Bei der 5. Verwendung wurden 3 ml Wasser zugesetzt.

### Ergebnisse:

| Verwendung | Std/Umsatz | g (S)-Mandelsäurenitril | % d. Theorie | % ee |
|---|---|---|---|---|
| 1. | 1/88 | 0,82 | 92 | 95,5 |
| 2. | 1/87 | 0,76 | 85 | 95,3 |
| 3. | 1,5/91 | 0,87 | 98 | 95,2 |
| 4. | 1,5/94 | 0,88 | 99 | 92,9 |
| 5. | 1/92 | 0,88 | 99 | 98,2 |
| 6. | 1/91 | 0,88 | 99 | 97,9 |
| 7. | 2/85 | 0,87 | 98 | 96,3 |

## Patentansprüche

1. Enantioselektives Verfahren zur Herstellung des (S)-Enantiomeren eines optisch aktiven Cyanhydrins durch Umsetzung eines Aldehyds oder eines Ketons mit einem Cyanidgruppendonor, dadurch gekennzeichnet, daß der Aldehyd oder das Keton in einem organischen Verdünnungsmittel in Gegenwart einer rekombinanten (S)-Hydroxynitrillyase aus Hevea brasiliensis mit einem Cyanidgruppendonor umgesetzt wird und das gebildete (S)-Cyanhydrin aus dem Reaktionsgemisch isoliert wird.

2. Verfahren nach Anspruch 1, dadurch gekennzeichnet, daß ein aliphatischer, aromatischer oder heteroaromatischer Aldehyd oder ein unsymmetrisches Keton umgesetzt wird.

3. Verfahren nach Anspruch 1, dadurch gekennzeichnet, daß als Cyanidgruppendonor Blausäure oder ein Cyanhydrin der Formel (R₁)(R₂)C(OH)(CN), in der R₁ und R₂ Alkylgruppen bedeuten, eingesetzt wird.

4. Verfahren nach Anspruch 3, dadurch gekennzeichnet, daß als Cyanidgruppendonor Blausäure oder Acetoncyanhydrin eingesetzt wird.

5. Verfahren nach Anspruch 1, dadurch gekennzeichnet, daß rekombinante (S)-Hydroxynitrillyase hergestellt aus Pichia pastoris oder Saccharomyces cerevisiae eingesetzt wird.

6. Verfahren nach Anspruch 1, dadurch gekennzeichnet, daß die (S)-Hydroxynitrillyase immobilisiert oder im Zweiphasen-System eingesetzt wird.

7. Verfahren nach Anspruch 1, dadurch gekennzeichnet, daß die (S)-Hydroxynitrillyase mehrfach wiederverwendet werden kann.

8. Verfahren nach Anspruch 1, dadurch gekennzeichnet, daß als organisches Verdünnungsmitel mit Wasser nicht mischbare aliphatische oder aromatische Kohlenwasserstoffe, Alkohole, Ether oder Ester verwendet werden.

9. Verfahren nach Anspruch 1, dadurch gekennzeichnet, daß als organisches Verdünnungsmittel Ethylacetat, Diisopropylether, Methyl-tert.butylether, Dibutylether verwendet werden.

## Claims

1. Enantioselective process for the preparation of the (S)-enantiomer of an optically active cyanohydrin by reaction of an aldehyde or of a ketone with a cyanide group donor, charcterized in that the aldehyde or the ketone is reacted with a cyanide group donor in an organic diluent in the presence of a recombinant (S)-hydroxynitrile lyase from Hevea brasiliensis and the (S)-cyanohydrin formed is isolated from the reaction mixture.

2. Process according to Claim 1, characterized in that an aliphatic, aromatic or heteroaromatic aldehyde or an unsymmetrical ketone is reacted.

3. Process according to Claim 1, characterized in that the cyanide group donor employed is hydrocyanic acid or a cyanohydrin of the formula (R₁) (R₂)C(OH) (CN), in which R₁ and R₂ are alkyl groups.

4. Process according to Claim 3, characterized in that the cyanide group donor is hydrocyanic acid or acetone cyanohydrin.

5. Process according to Claim 1, characterized in that recombinant (S)-hydroxynitrile lyase prepared from Pichia pastoris or Saccharomyces cerevisiae is employed.

6. Process according to Claim 1, characterized in that the (S)-hydroxynitrile lyase is immobilized or employed in a two-phase system.

7. Process according to Claim 1, characterized in that the (S)-hydroxynitrile lyase can be reused a number of times.

8. Process according to Claim 1, characterized in that the organic diluent used is a water-immiscible aliphatic or aromatic hydrocarbon, alcohol, ether or ester.

9. Process according to Claim 1, characterized in that the organic diluent used is ethyl acetate, diisopropyl ether, methyl tert-butyl ether or dibutyl ether.

## Revendications

1. Procédé énantiosélectif pour la préparation de l'énantiomère (S) d'une cyanhydrine optiquement active par réaction d'un aldéhyde ou d'une cétone avec un donneur de groupe cyanure, caractérisé en ce qu'on fait réagir l'aldéhyde ou la cétone dans un diluant organique en présence d'une (S)-hydroxynitrilase recombinante provenant de *Hevea brasiliensis* avec un donneur de groupe cyanure et on isole la (S)-cyanhydrine formée à partir du mélange de réaction.

2. Procédé selon la revendication 1, caractérisé en ce qu'on fait réagir un aldéhyde aliphatique, aromatique ou hétéroaromatique ou une cétone asymétrique.

3. Procédé selon la revendication 1, caractérisé en ce qu'on utilise, comme donneur de groupe cyanure, de l'acide cyanhydrique ou une cyanhydrine de formule (R₁)(R₂)C(OH)(CN) dans laquelle R₁ et R₂ signifient des groupes alkyle.

4. Procédé selon la revendication 3, caractérisé en ce qu'on utilise de l'acide cyanhydrique ou de l'acétonecyanhydrine comme donneur de groupe cyanure.

5. Procédé selon la revendication 1, caractérisé en ce qu'on utilise une (S)-hydroxynitrilase recombinante préparée à partir de *Pichia pastoris* ou de *Saccharomyces cerevisiae.*

6. Procédé selon la revendication 1, caractérisé en ce qu'on utilise la (S)-hydroxynitrilase à l'état immobilisé ou dans un système à deux phases.

7. Procédé selon la revendication 1, caractérisé en ce qu'on peut réutiliser plusieurs fois la (S)-hydroxynitrilase.

8. Procédé selon la revendication 1, caractérisé en ce qu'on utilise, comme diluant organique, des hydrocarbures aliphatiques ou aromatiques non miscibles avec l'eau, des alcools, des éthers ou des esters.

9. Procédé selon la revendication 1, caractérisé en ce qu'on utilise, comme diluant organique, de l'acétate d'éthyle, de l'éther diisopropylique, de l'éther méthyl-tertio-butylique ou de l'éther dibutylique.
